# EUROPÄISCHE PATENTSCHRIFT

(11) **EP 2 263 606 B1**
(45) Veröffentlichungstag und Bekanntmachung des Hinweises auf die Patenterteilung: **20.09.2017**
(21) Anmeldenummer: 10011732.4
(22) Anmeldetag: 02.08.1999
(51) Int. Cl.: A61F 2/06, A61F 2/91, A61F 2/915

(54) **Kompakter Stent**
Compact stent
Stent compacte

(30) Priorität: 05.09.1998 DE 19840645
(43) Veröffentlichungstag der Anmeldung: 22.12.2010
(62) Teilanmeldung aus: 99115265.3
(73) Patentinhaber: Abbott Laboratories Vascular Enterprises Limited, Dublin 2 (IE)
(72) Erfinder: von Oepen, Randolf, Dr., Los Altos, CA 94024 (US); Seibold, Gerd, 72119 Ammerbuch (DE)
(74) Vertreter: Hoefer & Partner Patentanwälte mbB

(56) Entgegenhaltungen:
- EP-A- 0 928 605
- WO-A-97/14375
- WO-A1-96/26689
- DE-U- 29 702 671

## Beschreibung

Die Erfindung betrifft einen Stent nach dem Oberbegriff des Anspruches 1.

Aus dem Stand der Technik sind unterschiedliche Ausgestaltungsformen von Stents bekannt. Diese bilden eine Gefäßprothese, welche aus körperverträglichem Material besteht. Stents werden im Allgemeinen dazu verwendet, Hohlgefäße, wie zum Beispiel Blutgefäße, oder auch Körperöffnungen aufzuweiten und in einem aufgeweiteten Zustand zu halten. Zu diesem Zweck wird der Stent normalerweise in einem nicht-expandierten Zustand im Körper des Patienten in ein verengtes Hohlgefäß positioniert und nachfol end durch geeignete Mittel, wie beispielsweise einen Ballonkatheter, aufgeweitet. Derartige Stents sind in der DE 297026714, der WO 96/26689 A1 und in der WO 97/14375 A1 beschrieben. Üblicherweise besteht der Stentkörper aus einer Stegstruktur, wobei die Stegstruktur mehrere zueinander benachbarte Stegmuster aufweist, die sich aneinander anreihende Stege aufweisen und die mittels Verbindungselementen miteinander verbunden sind.

Ein grundlegendes Problem bei vielen Stentkonstruktionen besteht darin, dass sie sich beim Aufweiten verkürzen. Die Verkürzung ist jedoch unerwünscht, da hierbei nicht ausgeschlossen werden kann, dass der aufgeweitete Stent aufgrund seiner Verkürzung den gesamten Bereich innerhalb des Gefäßes oder der Körperöffnung erfaßt, den er beispielsweise aufweiten und stützen soll, nicht mehr abdeckt.

Es ist daher Aufgabe der vorliegenden Erfindung, einen Stent der im Oberbegriff des Anspruchs 1 angegebenen Art zu schaffen, der im nicht-expandierten Zustand flexibel ist, im expandierten Zustand ausreichende Haltekräfte aufbaut, um in diesem Zustand zu verharren und dabei seine Länge beim Expandieren möglichst wenig verringert.

Die Lösung dieser Aufgabe erfolgt durch die Merkmale des Anspruches 1.

Dadurch, dass jeder der Stege der Stegmuster drei Teilbereiche aufweist, die im Winkel zueinander angeordnet sind, wird erreicht, dass beim Aufweiten die Winkel zwischen den Teilbereichen größer werden, was die Schrumpfung des Stents beim Aufweiten minimiert wenn nicht gar nahezu eliminiert.

Bei dieser Konstruktion weist der erfindungsgemäße Stent vorzugsweise im nicht-expandierten Zustand eine hohe Flexibilität auf, die seine Führbarkeit innerhalb des Gefäßes bis zur Implantationsstelle, beispielsweise im aufgekrimmten Zustand auf einem Katheter, sehr vorteilhaft beeinflußt. Ferner ermöglicht die erfindungsgemäße Konstruktion eine sehr stabile Konstruktion im aufgeweiteten Zustand, so dass der implantierte Stent hohe Kräfte aufnehmen kann und somit eine sehr gute Stützfunktion im implantierten Zustand ausüben kann.

Die Unteransprüche haben vorteilhafte Weiterbildungen der Erfindung zum Inhalt.

Vorzugsweise sind die Teilbereiche jedes Stegs geradlinig ausgebildet.

Ferner sind die Stege in einen mittleren Teilbereich und zwei Seiten-Teilbereiche unterteilt, die sich an die Enden des mittleren Teilbereiches anschließen. Hierbei nehmen die seitlichen Teilbereiche vorzugsweise stumpfe Winkel zum mittleren Teilbereich ein.

Die Anordnung der drei Teilbereiche zueinander ist hierbei vorzugsweise so getroffen, dass eine schüssel- oder tellerähnliche Konfiguration erreicht wird. Diese Konfiguration wiederum ermöglicht beim Zusammenkrimpen des Stents eine sehr kompakte Form, da sich die Stege vergleichbar ineinander gestapelten Tellern ineinanderlegen.

Die Stegmuster sind untereinander vorzugsweise durch Verbindungselemente miteinander verbunden, die als geradlinige Stege ausgebildet sind.

Die geradlinigen Stege verlaufen hierbei bei einer besonders bevorzugten Ausführungsform geradlinig in Verbindungsabschnitte der Stegmuster, die jeweils benachbarte Stege miteinander verbinden.

Die Ausrichtung der Verbindungselemente zwischen zwei benachbarten Stegmustern ist jeweils gleich. Das heißt, übereinander liegende Verbindungselemente haben jeweils die gleiche Ausrichtung. Andererseits ändern sich die Ausrichtungen der Verbindungselemente zwischen jeweils zwei benachbarten Stegmustern alternierend, so dass sie zum Beispiel bei Betrachtung einer in die Ebene abgewickelten Wand eines Stents abwechselnd eine Ausrichtung der Verbindungselemente einmal nach oben und einmal nach unten ergibt.

Der erfindungsgemäße Stent weist den besonderen Vorteil auf, dass er je nach Materialverwendung entweder als selbst-expandierender Stent oder als mittels eines Ballonkatheters aufweitbarer Stent ausgebildet werden kann. In beiden Fällen bleiben seine vorteilhaften zuvor erläuterten Eigenschaften erhalten. Wird ein selbst-expandierender Stent gewünscht, ist als Material vorzugsweise eine Nickel-Titan-Legierung zu verwenden.

Weitere Einzelheiten, Merkmale und Vorteile der Erfindung ergeben sich aus nachfolgender Beschreibung des Ausführungsbeispiels anhand der Zeichnungen.

Es zeigt:
- Fig. 1: eine stark vereinfachte perspektivische Darstellung des grundsätzlichen Aufbaus eines erfindungsgemäßen Stents;
- Fig. 2: eine schematisch leicht vereinfachte Darstellung eines Teiles der Stegstruktur der Wand des erfindungsgemäßen Stents im nichtexpandierten Zustand;
- Fig. 3: eine der Fig. 2 entsprechende Darstellung der Stegstruktur des erfindungsgemäßen Stents im aufgeweiteten Zustand;
- Fig. 4: eine vergrößerte Darstellung eines Teils der Stegstruktur des Stents im Zustand gemäß Fig. 2; und
- Fig. 5: eine der Fig. 3 entsprechende Darstellung einer zweiten Ausführungsform des erfindungsgemäßen Stents.

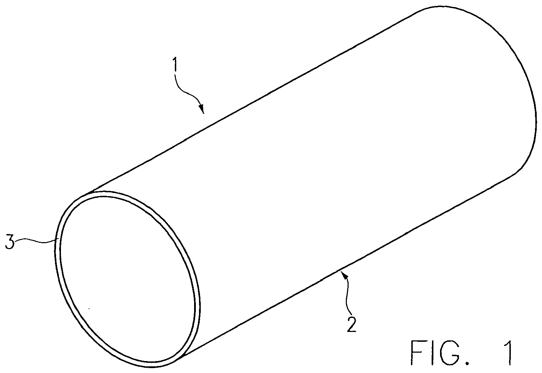

In Fig. 1 ist ein Stent 1 mit einem rohrförmigen flexiblen Körper 2 in perspektivischer schematisch vereinfachter Darstellung gezeigt.

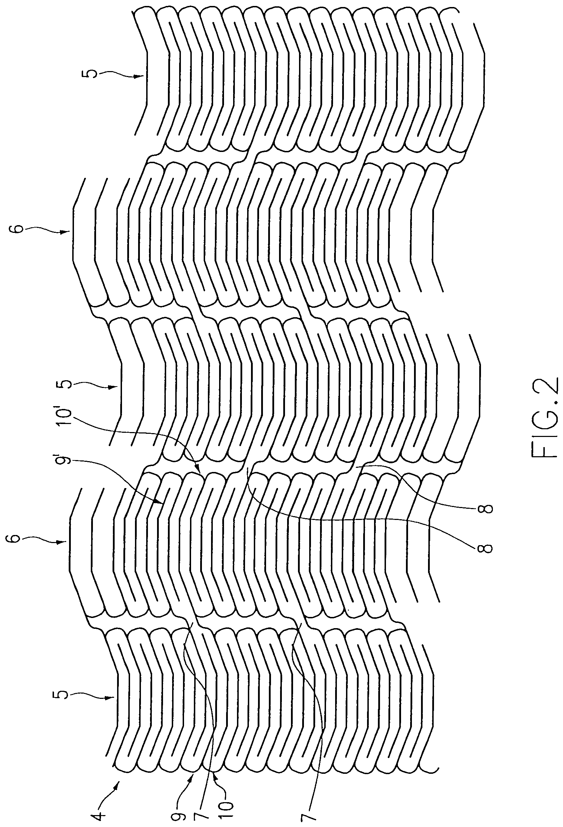

Der rohrförmige flexible Körper 2 wiederum weist eine Wand 3 mit einer Stegstruktur auf, die nachfolgend unter Bezugnahme auf die Fig. 2 bis 4 im einzelnen erläutert wird.

In Fig. 2 ist die Stegstruktur 4 im nicht-expandierten Zustand dargestellt.

Die Stegstruktur 4 weist benachbarte Stegmuster 5, 6 auf, die abwechselnd nebeneinander angeordnet sind, so dass die Stegmuster gemäß dem in Fig. 2 dargestellten Ausschnitt in der Reihenfolge 5, 6, 5, 6, 5, 6 usw. angeordnet sind. Fig. 2 verdeutlicht hierbei, dass die Stegmuster 5 und 6 sich aneinander anreihende Stege 9 und 10 aufweisen. Die Ausbildung dieser Stege 9, 10 wird nachfolgend noch genauer beschrieben, Fig. 2 verdeutlicht jedoch, dass die Stege 9, 10 eine teller- bzw. schüsselähnliche Ausbildung haben und sich gemäß der in Fig. 2 gewählten Darstellung nach oben öffnen.

Die Stege 9', 10' des benachbarten Stegmusters 6 haben die gleiche teller- bzw. schüsselförmige Ausbildung, öffnen sich jedoch gemäß Fig. 2 nach unten.

Die Stegmuster 5, 6 sind jeweils mittels Verbindungselementen 7 zwischen den Stegmustern 5 und 6 bzw. Verbindungselementen 8 zwischen den Stegmustern 6 und 5 miteinander verbunden. Fig. 2 verdeutlicht hierbei, dass jeweils eine Mehrzahl von Verbindungselementen 7 zwischen den Stegmustern 5 und 6 bzw. 8 zwischen den Stegmustern 6 und 5 vorgesehen sind, in Fig. 2 aufgrund des Ausschnittes jedoch jeweils nur zwei Verbindungselemente dargestellt sind. Die Verbindungselemente 7 haben hierbei alle die gleiche Ausrichtung, die gemäß der in Fig. 2 gewählten Darstellung von links unten nach rechts oben verläuft.

Die Verbindungselemente 8 haben ebenfalls die gleiche Ausrichtung untereinander, verlaufen jedoch gemäß der in Fig. 2 gewählten Darstellung (eine Abwicklung der Wand in die Ebene der Fig. 2) von links oben nach rechts unten. Diese Ausrichtung wechselt alternierend jeweils zwischen den Stegmustern 5, 6 bzw. 6, 5, wie sich dies aus Fig. 2 ergibt.

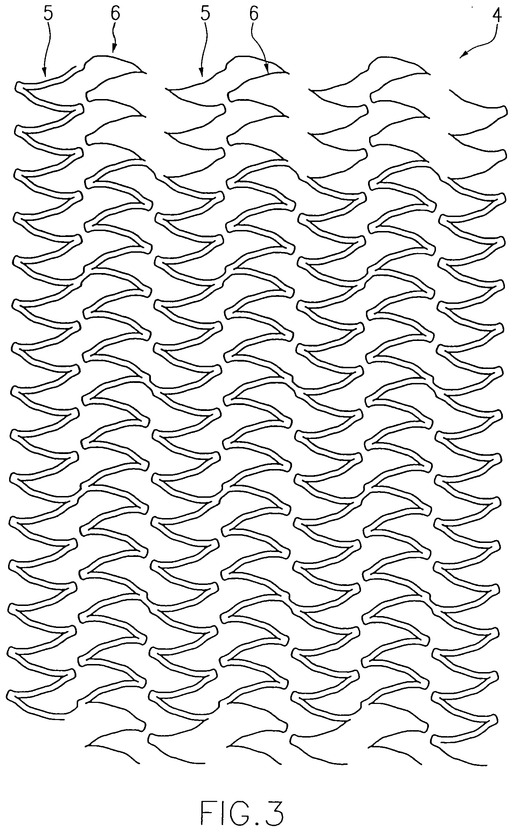

Fig. 3 verdeutlicht den expandierten Zustand des Stents 1 wiederum anhand eines Ausschnittes der Stegstruktur 4 in einer in die Ebene der Fig. 3 abgewickelten Darstellung der Wand 3 des Körpers 2 des Stents 1. Fig. 3 verdeutlicht hierbei die Spreizung der Stegstruktur 4, die dem Stent in der expandierten Stellung eine hohe Eigensteifigkeit verleiht, die das Verbleiben des Stents 1 in dieser expandierten Stellung ermöglicht und die Aufnahme von Radialkräften, wie sie beispielsweise bei der Implantierung des Stents 1 in ein Hohlgefäß im Bereich einer Stenose aufzunehmen sind.

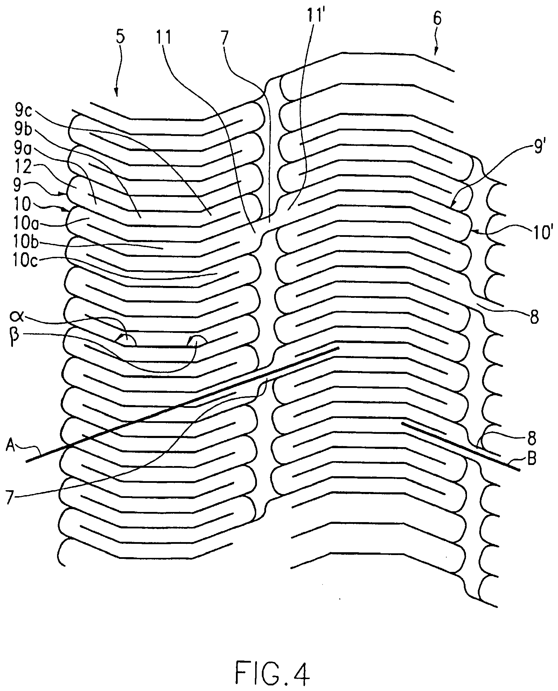

In Fig. 4 ist eine vergrößerte Darstellung eines Ausschnittes der Stegstruktur 4 im Zustand gemäß Fig. 2 dargestellt.

Fig. 4 verdeutlicht hierbei, dass die Stege 9, 10 jeweils drei Teilabschnitte 9a bis 9c bzw. 10a bis 10c aufweisen. Die Teilabschnitte 9a bis 9c sind jeweils geradlinig ausgebildet und schließen sich aneinander an, um die zuvor genannte teller- bzw. schüsselähnliche Konfiguration zu bilden. Die Teilbereiche 9a und 9b schließen hierbei einen stumpfen Winkel α ein. Der mittlere Teilbereich 9b und der rechte Teilbereich 9c schließen einen stumpfen Winkel β ein.

Entsprechend ist die Ausbildung der Teilabschnitte 10a bis 10c des sich an den Steg 9 anschließenden Steges 10, der bei der in Fig. 4 gewählten Darstellung unterhalb des Steges 9 liegt. Fig. 4 verdeutlicht hierbei, dass die Stege 9 und 10, die sich abwechselnd aneinander anschließen, jeweils wie ineinander gestapelte Teller im nicht-expandierten Zustand des Stents 1 angeordnet sind. Fig. 4 zeigt hierbei, dass die zuvor beschriebene Konfiguration der Teilabschnitte der Stege natürlich für jeden der Stege gilt, die zusammen den in Fig. 1 dargestellten rohrförmigen Zustand der Wand des Stents 1 mit der beschriebenen Stegstruktur bilden.

Untereinander sind die Stege 9, 10 jeweils über gerundete Verbindungsabschnitte 12 miteinander verbunden, von denen in Fig. 4 repräsentativ ein Verbindungsabschnitt 12 dargestellt ist.

Eine entsprechende Ausbildung gilt für die Stege 9', 10' des benachbarten Stegmusters 6.

Ferner zeigt Fig. 4 nochmals die Anordnung der Verbindungselemente 7, 8. Die Verbindungselemente 7 zwischen dem Stegmuster 5 und dem benachbarten Stegmuster 6 haben bei der in Fig. 4 gewählten Darstellung eine Ausrichtung A, die jeweils untereinander, also bei allen Verbindungselementen 7 die selbe ist. Die Ausrichtung A ist durch eine gerade Linie in Fig. 4 symbolisiert und verläuft gemäß Fig. 4 von links unten nach rechts oben.

Die Ausrichtung der Verbindungselemente 8 ist durch die Linie B dargestellt und verläuft von links oben nach rechts unten. Die Ausrichtung aller Verbindungselemente 8 untereinander ist jeweils gleich. Es ergibt sich mithin über die gesamte Stegstruktur eine alternierend sich ändernde Ausrichtung A, B, A, B usw.

Die Verbindungselemente 7, 8 sind jeweils als gerade Stege ausgebildet, die in einen Verbindungsabschnitt 11 des Stegmusters 5 bzw. 11' des Stegmusters 6 geradlinig übergehen, was in Fig. 4 anhand eines Verbindungselementes 7 mit seinen benachbarten Verbindungsabschnitten 11 bzw. 11' symbolisch für alle anderen Verbindungselemente 7 wie auch 8 dargestellt ist.

Durch die Ausbildung der Stege bestehend aus drei Teilabschnitten und den zwischen diesen angeordneten Winkeln α, β, die vorzugsweise stumpfwinklig sind, ergibt sich im in Fig. 3 dargestellten aufgespreizten Zustand eine Vergrößerung dieser Winkel α, β, die auf besonders vorteilhafte Weise die Kraftaufnahmefähigkeit des Stents in der aufgeweiteten Stellung ergibt. In der nicht-expandierten Stellung ist der Stent sehr flexibel, so dass er beim Hindurchführen durch Körpergefäße sich sehr gut an Krümmungen anpassen kann, so dass der Implantationsvorgang erheblich erleichtert wird.

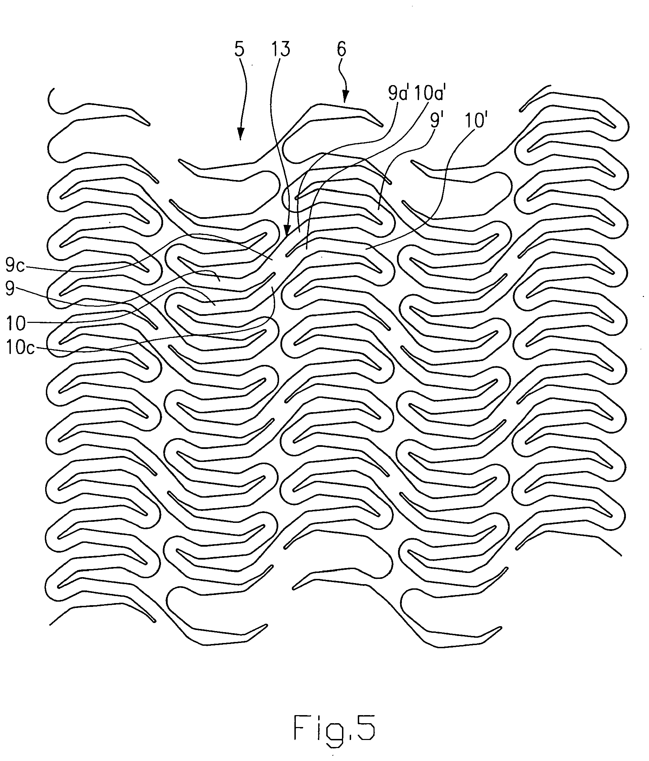

In Fig. 5 ist eine zweite Ausführungsform eines erfindungsgemäßen Stents entsprechend der Darstellung gemäß Fig. 3, also im aufgeweiteten Zustand dargestellt.

Die Grundkonstruktion dieser Ausführungsform entspricht derjenigen der zuvor erläuterten Ausführungsform. Dementsprechend handelt es sich bei dieser Ausführungsform ebenfalls um einen Stent mit einem rohrförmigen flexiblen Körper, dessen Wand eine Stegstruktur aufweist, die von einem nicht expandierten in einen in Fig. 5 dargestellten expandierten Zustand überführt werden kann.

Die Stegstruktur weist ebenfalls eine Vielzahl von benachbarten Stegmustern auf, von denen in Fig. 5 zwei exemplarisch mit den Bezugsziffern 5 und 6 gekennzeichnet sind. Die Stegmuster wiederum weisen sich aneinander anreihende Stege 9, 10 beziehungsweise 9', 10' auf. Jeder der Stege 9, 10 beziehungsweise 9', 10' ist ebenfalls in drei Teilabschnitte unterteilt, so dass diesbezüglich auf die voranstehende Erläuterung, insbesondere zu Fig. 4 verwiesen werden kann.

Die Ausführungsform gemäß Fig. 5 unterscheidet sich von der zuvor erläuterten Ausführungsform durch das Fehlen jeglicher Verbindungselemente zwischen den Stegmustern. Fig. 5 verdeutlicht, dass bei dieser Ausführungsform an vorbestimmbaren Übergangsabschnitten 13 die Stegmuster ineinander übergehen, wobei benachbarte Teilabschnitte entsprechender Stege, hier die Teilabschnitte 9c und 9'a beziehungsweise 10c und 10'a, verlängert sind und somit jeweils ein integraler Übergangsabschnitt 13 gebildet wird. Wie Fig. 5 zeigt, ergibt sich hieraus im Bereich der Übergangsabschnitte 13 eine asymmetrische Ausbildung der Stegmuster, wobei die Übergangsabschnitte 13 zur Erhöhung der Versteifung eine Dimensionierung D aufweisen, die größer ist als die Summe der Stegbreiten B1 und B2.

Aus Fig. 5 erschließt sich hierbei, dass pro benachbarter Stegmuster jedes dritte benachbarte Stegpaar 9, 9' beziehungsweise 10, 10' diesen integralen Übergangsabschnitt 13 aufweist. Grundsätzlich ist es jedoch auch möglich, eine größere Anzahl oder eine geringere Anzahl derartiger Übergangsabschnitte 13 vorzusehen.

Der besondere Vorteil dieser Ausführungsform ist eine äußerst kompakte Bauform bei gleich guter Flexibilität und Festigkeit im expandierten Zustand.

Ferner erläutert Fig. 5, dass die Übergangsabschnitte 13 ähnlich den Verbindungselementen 7 eine alternierende Ausrichtung haben, wozu wiederum auf die Ausführungsform gemäß Fig. 4 verwiesen werden kann. Ferner verdeutlicht Fig. 5, dass sich, insbesondere im expandierten Zustand, eine H-ähnliche Ausbildung des Übergangsabschnittes 13 mit den angrenzenden Steg-Teilabschnitten ergibt.

## Patentansprüche

1. Stent (1)
- mit einem rohrförmigen flexiblen Körper (2), dessen Wand (3) eine expandierbare Stegstruktur (4) aufweist,
- wobei die Stegstruktur (4) eine Vielzahl von benachbarten Stegmustern (5, 6) aufweist, die sich aneinander anreihende Stege (9, 10 bzw. 9', 10') aufweisen,
- wobei jeder Steg (9, 10) drei Teilbereiche (9a, 9b, 9c bzw. 10a, 10b, 10c) aufweist, die im Winkel (α, β) zueinander angeordnet sind,
- wobei ein mittlerer Teilbereich (9b bzw. 10b) vorgesehen ist, an dessen seitliche Enden sich jeweils ein Teilbereich (9a, 9c, bzw. 10 a, 10c) unter Einschluss eines stumpfen Winkels (α, β) anschließt,
- wobei die Stegmuster (5, 6) an vorbestimmbaren integralen Übergangsabschnitten (13) ineinander übergehen,
- wobei die Ausrichtung aller Übergangsabschnitte (13) zwischen unmittelbar benachbarten Stegmustern (5, 6) bzw. (6, 5) gleich ist, **dadurch gekennzeichnet,**
- **dass** sich, über die gesamte Stegstruktur (4), die Ausrichtung (A, B) der Übergangsabschnitte (13) alternierend ändert.

2. Stent nach Anspruch 1, **dadurch gekennzeichnet, dass** jeder Teilbereich (9a, 9b, 9c bzw. 10a, 10b, 10c) geradlinig ist.

3. Stent nach Anspruch 1 oder 2, **dadurch gekennzeichnet, dass** die Übergangsabschnitte (13) durch Verlängerungen benachbarter Teilabschnitte (9c, 9a' beziehungsweise 10c, 10a') aneinander angrenzender Stege (9, 10 beziehungsweise 9', 10') gebildet sind.

4. Stent nach einem der Ansprüche 1 bis 3, **dadurch gekennzeichnet, dass** die Übergansabschnitte (13) eine Dimensionierung D aufweisen, die größer ist als die Summe der Stegbreiten (B1, B2).

5. Stent nach einem der Ansprüche 1 bis 4, **dadurch gekennzeichnet, dass** sich eine H-ähnliche Ausbildung des Übergangsabschnittes (13) mit den angrenzenden Steg-Teilabschnitten (9c, 10c, 9a', 10a') ergibt.

6. Stent nach einem der Ansprüche 1 bis 5, **dadurch gekennzeichnet, dass** pro benachbarter Stegmuster (5, 6) jedes dritte benachbarte Stegpaar (9, 9' bzw. 10, 10') einen Übergansabschnitt (13) aufweist.

7. Stent nach einem der Ansprüche 1 bis 5, **dadurch gekennzeichnet, dass** pro benachbarter Stegmuster (5, 6) mehr als jedes dritte benachbarte Stegpaar (9, 9' bzw. 10, 10') einen Übergansabschnitt (13) aufweist.

8. Stent nach einem der Ansprüche 1 bis 5, **dadurch gekennzeichnet, dass** pro benachbarter Stegmuster (5, 6) weniger als jedes dritte benachbarte Stegpaar (9, 9' bzw. 10, 10') einen Übergansabschnitt (13) aufweist.

9. Stent nach einem der Ansprüche 1 bis 8, **dadurch gekennzeichnet, dass** die Stegstruktur (4) der Wand (3) aus einer Nickel-Titan-Legierung besteht.

10. Stent nach einem der Ansprüche 1 bis 9, **dadurch gekennzeichnet, dass** das Material der Wand (3) körperverträglich ist.

## Claims

1. A stent (1) comprising a tubular flexible body (2) with a wall (3) comprising an expandable web structure (4),
wherein the web structure (4) comprises a plurality of neighboring web patterns (5, 6), each web pattern comprising adjoining webs (9, 10; 9', 10'),
wherein each web (9, 10) comprises three sections (9a, 9b, 9c; 10a, 10b, 10c) arranged relative to one another at an angle (α, β),
wherein a central section (9a; 10b) is provided, the central section intersecting adjoining end sections at an obtuse angle (α, β),
wherein the web patterns (5, 6) pass into each other at predeterminable integral transition sections (13),
wherein the transition sections (13) of directly neighboring web patterns (5, 6); (6, 5) have a common orientation (A, B),
**characterized in that** the orientation (A, B) of the transition sections (13) is alternating over the entire web structure (4).

2. Stent according to claim 1 **characterized in that** each one of the three interconnected sections (9a, 9b, 9c; 10a, 10b, 10c) is straight.

3. The stent according to claim 1 or claim 2, **characterized in that** said transition sections (13) are formed by extensions of neighboring sections (9c, 9'a); (10c, 10'a) of adjoining webs (9, 10); (9', 10').

4. The stent according to any of claims 1 to 3, **characterized in that** the transition sections (13) comprise a dimension D greater than the sum of the web widths (B1, B2).

5. Stent according to any of claims 1 to 4, **characterized in that** an H-like configuration of the transition section (13) with the adjoining web sections (9c, 10c, 9a', 10a') is obtained.

6. Stent according to any of claims 1 to 5, **characterized in that** per neighboring web pattern (5, 6) every third neighboring pair of webs (9, 9'); (10, 10') comprises an integral transition section (13).

7. Stent according to any of claims 1 to 5, **characterized in that** per neighboring web pattern (5, 6) more than every third neighboring pair of webs (9, 9'); (10, 10') comprises an integral transition section (13).

8. Stent according to any of claims 1 to 5, **characterized in that** per neighboring web pattern (5, 6) less than every third neighboring pair of webs (9, 9'); (10, 10') comprises an integral transition section (13).

9. The stent according to any of claims 1 to 8, **characterized in that** the web structure (4) of the wall (3) is made from a nickel-titanium alloy.

10. The stent according to any of claims 1 to 9, **characterized in that** the wall material (3) is bio-compatible.

## Revendications

1. Stent (1),
- comprenant un corps (2) flexible de forme tubulaire, dont la paroi (3) présente une structure d'entretoise (4) expansible,
- dans lequel la structure d'entretoise (4) présente une pluralité de motifs d'entretoise (5, 6) adjacents, que les entretoises (9, 10 ou 9', 10') alignées les unes avec les autres présentent,
- dans lequel chaque entretoise (9, 10) présente trois zones partielles (9a, 9b, 9c ou 10a, 10b, 10c), qui sont disposées les unes par rapport aux autres selon l'angle (α, β),
- dans lequel une zone partielle (9b ou 10b) centrale est prévue, aux extrémités latérales de laquelle se raccorde respectivement une zone partielle (9a, 9c ou 10a, 10c) en formant un angle obtus (α, β),
- dans lequel les motifs d'entretoise (5, 6) s'imbriquent les uns dans les autres au niveau de sections de transition (13) intégrales pouvant être prédéfinies,
- dans lequel l'orientation de toutes les sections de transition (13) entre des motifs d'entretoise (5, 6) ou (6, 5) directement adjacents est identique, **caractérisé en ce**
- **que** l'orientation (A, B) des sections de transition (13) varie en alternance sur l'ensemble de la structure d'entretoise (4).

2. Stent selon la revendication 1, **caractérisé en ce que** chaque zone partielle (9a, 9b, 9c ou 10a, 10b, 10c) est rectiligne.

3. Stent selon la revendication 1 ou 2, **caractérisé en ce que** les sections de transition (13) sont formées par des prolongements de sections partielles (9c, 9a' ou 10c, 10a') adjacentes d'entretoises (9, 10 ou 9', 10') se jouxtant les unes les autres.

4. Stent selon l'une quelconque des revendications 1 à 3, **caractérisé en ce que** les sections de transition (13) présentent une dimension D, qui est plus grande que la somme des largeurs d'entretoise (B1, B2).

5. Stent selon l'une quelconque des revendications 1 à 4, **caractérisé en ce qu'**il en résulte une réalisation similaire à un H de la section de transition (13) avec les sections partielles d'entretoise (9c, 10c, 9a', 10a') se jouxtant les unes les autres.

6. Stent selon l'une quelconque des revendications 1 à 5, **caractérisé en ce que** par motif d'entretoise (5, 6) adjacent, chaque troisième paire d'entretoises (9, 9' ou 10, 10') adjacente présente une section de transition (13).

7. Stent selon l'une quelconque des revendications 1 à 5, **caractérisé en ce que** par motif d'entretoise (5, 6) adjacent, plus d'une paire d'entretoises (9, 9' ou 10, 10') adjacente sur trois présente une section de transition (13).

8. Stent selon l'une quelconque des revendications 1 à 5, **caractérisé en ce que** par motif de stent (5, 6) adjacent, moins d'une paire d'entretoises (9, 9' ou 10, 10') sur trois présente une section de transition (13).

9. Stent selon l'une quelconque des revendications 1 à 8, **caractérisé en ce que** la structure d'entretoise (4) de la paroi (3) est constituée d'un alliage de nickel et de titane.

10. Stent selon l'une quelconque des revendications 1 à 9, **caractérisé en ce que** le matériau de la paroi (3) est toléré par le corps.
